(19) Europäisches Patentamt
European Patent Office
Office européen des brevets

(11) Veröffentlichungsnummer: **0 371 195 B1**

(12) **EUROPÄISCHE PATENTSCHRIFT**

(45) Veröffentlichungstag der Patentschrift: **29.09.93**

(51) Int. Cl.5: **A61K 47/48**, A61K 37/26, A61K 37/02, A61K 9/02

(21) Anmeldenummer: **89112726.8**

(22) Anmeldetag: **12.07.89**

Die Akte enthält technische Angaben, die nach dem Eingang der Anmeldung eingereicht wurden und die nicht in dieser Patentschrift enthalten sind.

(54) **Verfahren zur Herstellung von zur rektalen und vaginalen Anwendung geeigneten Präparaten biologisch aktiver Peptide.**

(30) Priorität: **12.07.88 DD 317829**

(43) Veröffentlichungstag der Anmeldung:
**06.06.90 Patentblatt 90/23**

(45) Bekanntmachung des Hinweises auf die Patenterteilung:
**29.09.93 Patentblatt 93/39**

(84) Benannte Vertragsstaaten:
**DE ES FR GB IT SE**

(56) Entgegenhaltungen:
DD-A- 252 539
DD-A- 254 881

RESEARCH DISCLOSURE, Nr. 269, September 1986, September 1986, Seite 26908, Emsworth, Hampshire, GB; "Rektale und vaginale Applikation von Gyrase-Inhibitoren"

(73) Patentinhaber: **VEB Berlin-Chemie**
**Glienicker Weg 125-127**
**D-12489 Berlin(DE)**

(72) Erfinder: **Kossowicz, Joachim**
**Kalkbergeweg 79**
**DDR-1165 Berlin(DD)**
Erfinder: **Hacker, Elke, Dr. med. vet.**
**Ahornstrasse 32**
**DDR-1167 Berlin(DD)**
Erfinder: **Milde, Karl, Dr. rer. nat.**

**Verstorben(DD)**
Erfinder: **Losse, Günter, Prof. Dr. sc. nat.**
**Zeunerstrasse 83**
**DDR-8027 Dresden(DD)**
Erfinder: **Müller, Frank**
**Franz-Lehmann-Strasse 8**
**DDR-8030 Dresden(DD)**

(74) Vertreter: **Hansen, Bernd, Dr.rer.nat. et al**
**Hoffmann, Eitle & Partner, Patentanwälte,**
**Postfach 81 04 20**
**D-81904 München (DE)**

**Beschreibung**

Die Erfindung betrifft ein Verfahren zur Herstellung von Präparaten zur Anwendung von biologisch aktiven Peptiden auf rektalem und vaginalem Wege in der Human- und Veterinärmedizin.

Seit der Einführung der Insulintherapie wird nach geeigneten Applikationsformen gesucht, die eine bedarfsgerechte Zuführung des Insulins in hoher Bioverfügbarkeit unter Umgehung des üblichen parenteralen Weges ermöglichen.

Der Einsatz von Resorptionsvermittlern (DD 117 681) liess die nasale und rektale Anwendung von Insulinpräparationen möglich werden.

Aus der DD-A- 254 881 ist ein Verfahren zur Herstellung von nicht parenteral, vorzugsweise rektal, applizierbaren Insulinpräparaten durch Einbettung, Einschluß oder Absorption des Insulins in gelartige makromolekulare, biologisch abbaufähige Trägermaterialien, inbesondere in Polysaccharid-, Polyamid- oder Polypeptidmatrices, z.B. Agar, bekannt, welche als Resorptionsvermittler ein Eiweißfettsäure-Kondensat und als Proteaseinhibitor Trypsininhibitor enthalten.

Jedoch ist bei alleiniger Anwendung von Resorptionsvermittlern auch unter Zusatz von Proteaseninhibitoren gegen Proteasen des Pankreas (WP DD 254 881) und der Steuerung der Liberationsgeschwindigkeit des Insulins aus einem polymeren Träger in einem günstigen Verhältnis zur Resorptionsgeschwindigkeit (WP DD 252 539, DD 254 881, DD 257 197) noch immer eine erhebliche Differenz in der Bioverfügbarkeit des Insulins gegenüber der Insulininjektion gegeben. Dies ist mit erheblichen ökonomischen Verlusten verbunden. Darin ist die Ursache zu sehen, dass bisher noch kein klinisch verwendbares Ergebnis erreicht wurde.

Die Probleme der Unbeständigkeit von Insulin im Gastro-Intestinal-Trakt einerseits und die schlechte Resorbierbarkeit so grosser polarer Moleküle andererseits treten auch bei diversen anderen biologisch aktiven Peptiden auf.

Es wurde versucht, sowohl Resorbierbarkeit als auch Stabilität gegen enzymatischen Abbau durch strukturelle Abwandlung des Moleküls zu beeinflussen. Dabei wird in der Regel die beabsichtigte Wirkqualität des Peptides verändert. Der Einsatz von Resorptionsvermittlern und der Schutz des Peptides vor enzymatischem Abbau durch Proteaseninhibitoren sind jeweils für sich beschrieben. Dies gilt auch für die gesteuerte Liberation durch Fixierung des Peptides an einen Träger.

Die Stabilität von Polypeptiden wie Insulin, aber auch für Oligopeptide und damit die Bioverfügbarkeit dieser Peptide bei der rektalen und vaginalen Resorption kann auch durch gegen anaerobe Bakterien wirkende Mittel positiv beeinflusst werden.

Jede der beschriebenen Möglichkeiten für sich allein stellt aber keine umfassende Lösung des Problems der unzureichenden Bioverfügbarkeit von Peptiden bei rektaler und vaginaler Applikation dar. Erst die erfindungsgemässe Kombination der genannten Möglichkeiten in einer pharmazeutischen Zubereitung gestattet eine ökonomisch sinnvolle rektale oder vaginale Resorption von Peptidwirkstoffen.

Ziel der Erfindung ist es, ein Verfahren zu offenbaren, das die Herstellung von Präparaten ermöglicht, mit denen biologisch aktive Peptide rektal bzw. vaginal appliziert werden können, wobei eine anhaltende Wirkung mit einer gleichbleibenden hohen Wirksamkeit des eingesetzten Peptides gewährleistet werden soll.

Der Erfindung liegt die Aufgabe zugrunde, ein neues Verfahren zur Herstellung von Präparaten zur Applikation biologisch aktiver Peptide unter Verwendung an sich bekannter Einzelelemente, die einen Kombinationseffekt ergeben, aufzuzeigen.

Die Aufgabe der Erfindung wird gelöst durch ein Verfahren, nach dem biologisch aktive Peptide, wie Insulin, GnRH und -Analoga, Substanz P und Derivate, Oligopeptide, wie Thymuspeptide und Analoga, aber auch mikrobiologisch hergestellte Peptide, wie Cyclosporine, an einen hochmolekularen inerten Träger fixiert, verlangsamt kontinuierlich freigesetzt werden, durch einen anionischen Resorptionsvermittler wie Aminosäure-, Peptid- oder Eiweiss-Fettsäure-Kondensat die Schleimhautpassage ermöglicht wird und durch die kombinierte Mitverwendung eines Proteaseninhibitors und eines Wirkstoffs gegen anaerobe Bakterien die biologische Aktivität des Peptides und die Wirkung des Resorptionsvermittlers über die Dauer der verlangsamten Freisetzung unter den Bedingungen einer rektalen/vaginalen Applikation voll erhalten wird.

Die Trägerfixierung des biologisch aktiven Peptides erfolgt nach DD-A-219 673; DD-A-257 197.

Der anionische Resorptionsvermittler wird durch Kondensation von Aminosäure bzw. Eiweisshydrolysat mit Fettsäure gewonnen (BONDI, S. Biochem. Z. 17/1909/545; IZAR, G. Biochem. Z. 40/1912/402; DD 106 783; DD 117 681). Die auf diesem Wege gewonnenen hochreinen Resorptionsvermittler sind schleimhautverträglich und biologisch abbaubar.

Um den biochemischen Abbau sowohl des bioaktiven Peptides als auch des Resorptionsvermittlers über die Dauer der verlangsamten Freisetzung im Bereich von Rektum bzw. Vagina zu verhindern, ist die

2

Gegenwart einer Kombination aus Proteaseninhibitor und einem Stoff, der die Wirkung der im Applikationsmilieu tätigen anaeroben Bakterien minimiert, wichtig. Als Proteaseninhibitor finden z.B. Aprotinin oder EAC (ε-Aminocapronsäure) Anwendung. Als Substanzen gegen anaerob existente Bakterien gelangen im Rahmen der Erfindung Chemotherapeutika und/oder Desinfektionsmittel, wie z.B. p-Hydroxybenzoesäureester, zum Einsatz.

Überraschend an der gefundenen Lösung war, dass die Bioverfügbarkeit der Peptide in der genannten Applikationsform nicht nur der Summierung der erreichbaren Einzelmerkmale entsprach, sondern weit darüber hinausgeht. Während sich die Bioverfügbarkeit von Peptiden durch eine Addition der Einzelmerkmale auf ca. 30 % erhöhen würde, wurde beim erfindungsgemässen Verfahren eine Bioverfügbarkeit von 60 bis 75 % gegenüber einer i.m.-Applikation festgestellt.

Ausführungsbeispiele

Beispiel 1

20,000 g Gelatine werden in einem Gemisch aus 25,000 g Glycerol, 19,0 ml Wasser und 4,0 ml 2 n NaOH 60 Minuten bei Raumtemperatur gequollen, bei 60 °C homogenisiert und nach Abkühlen auf 40 °C 20,0 g Insulin-Agar-Addukt 1,000 g Eiweiss-Fettsäure-Kondensat, 5,0 g Nipagin® und 150.000 ATrE Aprotinin homogen eingerührt. Anschliessend wird in Formen gegossen.

Beispiel 2

20,000 g eines Insulin-Agar-Adduktes mit 5 IE Insulin pro 200 mg Addukt werden gemeinsam mit 0,240 g Lauroylleucin, 6 mg Metronidazol und 150.000 ATrE Aprotinin homogen in einer geschmolzenen Suppositorienmasse des Types Rosupol® R verteilt und in Suppositorienformen zu je 2,0 g gegossen. Nach Erkalten wird ausgeformt.

Beispiel 3

20,000 g eines Insulin-Agar-Adduktes mit 2,4 IE Insulin pro 200 mg Addukt werden gemeinsam mit 0,240 g Lauroylleucin, 250 mg Metronidazol und 150.000 ATrE Aprotinin homogen in einer geschmolzenen Suppositorienmasse des Types Rosupol® R verteilt, in Glaskapillaren aufgenommen und sofort auf 0 °C abgekühlt. Die so entstandenen Zylinder werden für die Applikation an diabetischen Ratten eingesetzt.

Beispiel 4

Ein 55%iger Insulin-Polyglycin-Komplex wurde durch Suspendieren von 250 mg Polyglycin ($\overline{M}$ 4000) in 7,5 ml 85%iger Ameisensäure unter Zugabe von 1,1 ml Insulinlösung (250 mg Insulin/ml 85%iger Ameisensäure) hergestellt. 5,7 mg dieses Komplexes wurden dem Gießansatz (7,5 g Glycerol, 8,9 Citrat-Phosphat-Puffer, pH 7,5, 9 g Gelatine) zugesetzt. Weiterhin wurden analog zu Beispiel 2 Resorptionsvermittler, Konservierungsmittel und Proteaseninhibitor hinzugefügt. Diese Masse wurde zu 2g-Suppositorien vergossen.

Bei einer Insulindosierung von 1,2 IE/kg Körpermasse ergab sich eine von 1 bis 4 Stunden anhaltende Blutglucosesenkung um 40 %. Das Ausgangsniveau wurde nach 6 Stunden erreicht.

Die Kontrolltiere, die nur Insulinzäpfchen mit Resorptionsvermittler ohne Fixierung des Insulins an Polyglycin erhielten, zeigten nur eine 1 bis 2 Stunden anhaltende Blutglucosesenkung von 30 %.

Beispiel 5

Analog zu Beispiel 4 wurden 11,4 mg eines 55%igen Insulin-Polyglycin-Komplexes mit 300 mg Lauroylleucin zu Zäpfchen so verarbeitet, dass 2g-Suppositorien mit 10 IE Insulin erhalten wurden. Nach rektaler Applikation einer Insulindosis von 2,5 IE/kg Körpermasse zeigten die Versuchstiere eine 4 Stunden anhaltende Blutglucosesenkung um 50 %. Das Ausgangsniveau wurde nach 7 Stunden erreicht.

Beispiel 6

Zur Herstellung eines 55%igen Insulin-α-Polylysin-Komplexes wurden 450 mg Polylysin ($\overline{M}$ = 4000) in 20 ml destilliertem Wasser gelöst und der Lösung 560 mg Insulin zugesetzt. 11,4 mg des so erhaltenen Komplexes wurden zusammen mit 300 mg Laurolylleucin und unter Zusatz von 100 mg Methylhydroxybenzoat sowie 150.000 ATrE Aprotinin wie unter Beispiel 4 zu 2g-Suppositorien verarbeitet. Aus einer Insulindosierung von 2,5 IE/kg Körpermasse zeigten die Versuchstiere eine 60%ige Blutglucosesenkung über 3 Stunden, wobei das Ausgangsniveau nach 6 Stunden wieder erreicht wurde.

Beispiel 7

1,8 g Sephadex G-100 und 7,2 ml destilliertes Wasser werden homogenisiert und 24 Stunden stehen gelassen. Danach wurde 1 ml Insulinlösung (250 mg/ml, 0,2 mol/l $Na_2HPO_4$-Lösung, pH 9,7) zugesetzt, 1 Stunde gut gerührt und 8 Stunden stehen gelassen. Anschliessend wird in 200 ml absolutem Aceton gefällt, abgesaugt und 36 Stunden über $P_2O_5$ bis zur Gewichtskonstanz getrocknet (130 Pa (1 Torr), 25 °C).

Die so erhaltenen Gele mit einem Gehalt von 10 IE/3,4 mg Sephadex wurden wie folgt weiterverarbeitet:

49,415 g Rosupol® wurden zusammen mit 500 mg Lauroylleucin bei 40 °C versetzt, 85 mg des Insulin-Sephadex-Adduktes zugesetzt und zu Suppositorien vergossen. Bei einer Insulindosierung von 10 IE/Suppositorium ergab sich eine Blutglucosesenkung um 50 % mit Erreichen des Ausgangsniveaus nach 6,5 Stunden.

Biologische Testergebnisse

1. Material und Methode

Kaninchen

Die Untersuchung erfolgte an männlichen stoffwechselgesunden Kaninchen mit einer Lebendmasse von 3,5 - 4,0 kg der Kreuzung Deutsche Widder - Chinchilla. Die Tiere waren auf normale Insulinempfindlichkeit vorgetestet worden.

Die Tiere erhielten 18 Stunden vor Versuchsbeginn kein Futter, Wasser jedoch ad libitum. Die Haltung erfolgte in Hausdrahtkäfigen, einstreulos, bei 100 g Standardpelletfutter/Tag und einer Raumtemperatur von 20 °C ± 2 °C ohne Helldunkel-Regime. Spezielle Versuchsanordnung siehe Arzneibuch der DDR.

Die Applikation erfolgte subkutan unter die Nackenhaut. Pro Versuchsgruppe wurden 10 Tiere eingesetzt. 10 Tiere bildeten die Kontrollgruppe (Gruppe 1). Die Blutentnahme erfolgte durch Punktion der Ohrvene. Die Glucosebestimmung wurde mittels Fermognost-Testbesteck durchgeführt.

Mäuseovulationstest

Infantile, 3 Wochen alte Mäuse erhalten 0,5 IE PMSG (pregnant mare serum gonadotropin) und 53 bis 54 Stunden später die ovulationsauslösende Zweitinjektion s.c., 18 bis 19 Stunden darauf erfolgt die Tötung und mikroskopische Untersuchung auf das Vorliegen ovulierter Eizellen im Oviduct.

| | |
|---|---|
| Injektionsvolumen: | jeweils 0,2 ml |
| Lösungsmittel: | physiologische NaCl-Lösung |
| Mäuseanzahl/Gruppe: | 9 bis 11 |

Die mathematische Berechnung der mittleren effektiven Dosis 50 (ED 50) erfolgte mittels Probilanalyse.

2. Plasmaglucose - Reduktionszeitprofil

2.1 Testergebnisse zu Beispiel 1, Versuchstiere: normoglykämische Kaninchen

| t | 20′ | 40′ | 60′ | 80′ | 120′ | 180′ | 240′ | 300′ |
|---|---|---|---|---|---|---|---|---|
| $\overline{x}$ | 58.6 | 49.3 | 52.3 | 65.8 | 78.4 | 84.3 | 92.7 | 90.9 |
| $1 \pm \overline{sx}$ | 15.3 | 9.72 | 11.12 | 15.7 | 19.3 | 20.63 | 18.77 | 15.69 |
| $\overline{x}$ | 55.3 | 52.9 | 44.7 | 45.2 | 53.7 | 56.5 | 67.2 | 76.8 |
| $2 \pm \overline{sx}$ | 13.62 | 11.59 | 13.72 | 9.36 | 14.84 | 17.61 | 14.3 | 18.8 |

1: Gelatinezäpfchen mit 5 IE Insulin-Agar-Addukt + 5 % Eiweiss-Fettsäure-Kondensat 2: Gelatinezäpfchen entsprechend Beispiel 1

2.2 Testergebnisse zu Beispiel 2, Versuchstiere: normoglykämische Kaninchen

| t | 20' | 40' | 60' | 80' | 120' | 180' | 240' | 300' |
|---|---|---|---|---|---|---|---|---|
| $\overline{x}$ | 65.5 | 50.5 | 50.6 | 46.7 | 63.5 | 80.4 | 91.5 | 86.6 |
| $1 \pm \overline{sx}$ | 19.57 | 12.34 | 9.03 | 13.57 | 18.20 | 17.63 | 19.58 | 19.03 |
| $\overline{x}$ | 63.4 | 55.3 | 50.3 | 42.3 | 51.9 | 50.3 | 50.6 | 47.9 |
| $2 \pm \overline{sx}$ | 18.20 | 15.73 | 12.80 | 15.70 | 13.50 | 16.80 | 18.40 | 20.30 |

1: 2g-Rosupolzäpfchen mit 5 IE Insulin-Agar-Addukt und $7,5 \times 10^{-5}$ Mol Lauroylleucin (1,2 %) 2: 2g-Rosupolzäpfchen entsprechend Beispiel Nr. 2

2.3 Testergebnisse zu Beispiel 3, Versuchstiere: diabetische Ratte

| t | 10′ | 20′ | 30′ | 40′ | 50′ | 60′ | 80′ |
|---|---|---|---|---|---|---|---|
| $\overline{x}$ | 88.93 | 65.37 | 57.68 | 52.69 | 48.73 | 51.91 | 46.28 |
| $1 \pm \overline{sx}$ | 11.32 | 12.32 | 10.9 | 9.05 | 10.76 | 12.15 | 10.01 |
| $\overline{x}$ | 88.33 | 67.00 | 55.51 | 47.82 | 42.16 | 44.14 | 36.15 |
| $2 \pm \overline{sx}$ | 9.72 | 12.77 | 11.88 | 10.92 | 9.82 | 9.97 | 6.29 |

1: 2,4 IE Insulin-Agar-Addukt/Zäpfchen + $7,5 \times 10^{-5}$ Mol N-Lauroylleucin (1,2 %) 2: Beispiel 1, aber 2,4 IE Insulin-Agar-Addukt/Zäpfchen n = 8

| t | 100′ | 120′ | 135′ | 150′ | 180′ | 210′ |
|---|---|---|---|---|---|---|
| $\overline{x}$ | 57.37 | 75.53 | 82.03 | 91.52 | 110.27 | 103.62 |
| $1 \pm \overline{sx}$ | 15.26 | 17.02 | 15.68 | 19.25 | 21.34 | 24.63 |
| $\overline{x}$ | 41.67 | 38.55 | 45.71 | 54.88 | 56.71 | 57.66 |
| $2 \pm \overline{sx}$ | 18.57 | 13.53 | 17.95 | 25.57 | 27.96 | 29.40 |

1: 2,4 IE Insulin-Agar-Addukt/Zäpfchen + $7,5 \times 10^{-5}$ Mol N-Lauroylleucin (1,2 %) 2: Beispiel 1, aber 2,4 IE Insulin-Agar-Addukt/Zäpfchen n = 8

2.4 Testergebnisse zu Beispiel Nr. 4

| | 20' | 40' | 60' | 80' | 100' | 120' | 180' | 240' | 300' | 360' |
|---|---|---|---|---|---|---|---|---|---|---|
| $\bar{x}$ | 100.55 | 84.82 | 72.51 | 71.69 | 71.38 | 82.11 | 85.97 | 93.9 | 94.92 | 98.10 |
| 1 ± $\bar{sx}$ | 17.32 | 13.26 | 12.43 | 14.59 | 15.08 | 10.88 | 10.91 | 11.37 | 14.01 | 12.03 |
| $\bar{x}$ | 82.56 | 68.32 | 62.31 | 58.92 | 57.32 | 63.25 | 67.79 | 69.08 | 78.92 | 92.18 |
| 2 ± $\bar{sx}$ | 12.46 | 14.01 | 11.01 | 8.96 | 10.32 | 15.77 | 14.32 | 10.77 | 15.61 | 12.31 |

1: 2g-Gelatinezäpfchen mit 5 IE Insulin/Supp. und 5 % Eiweiss-Fettsäure-Kondensat 2: 2g-Gelatinezäpfchen entsprechend Beispiel Nr. 4

3. Vergleich der Flächenintegrale der Beispiele 1 bis 4 (Tabelle 1)

Tabelle 1: AUC: (Flächenintegrale) der Glucoseprofile in $mm^2$ der Beispiele 1 bis 3

| Beispiel | gemäss Vergleichs-rezeptur | gemäss Erfin-dung | % Wirkungs-steigerung |
|---|---|---|---|
| | AUC: in $mm^2$ | | |
| 1 | 3721 | 6910 | 85 |
| 2 | 4643 | 8094 | 75 |
| 3 | 3034 | 5740 | 90 |
| 4 | 2238 | 5319 | 138 |

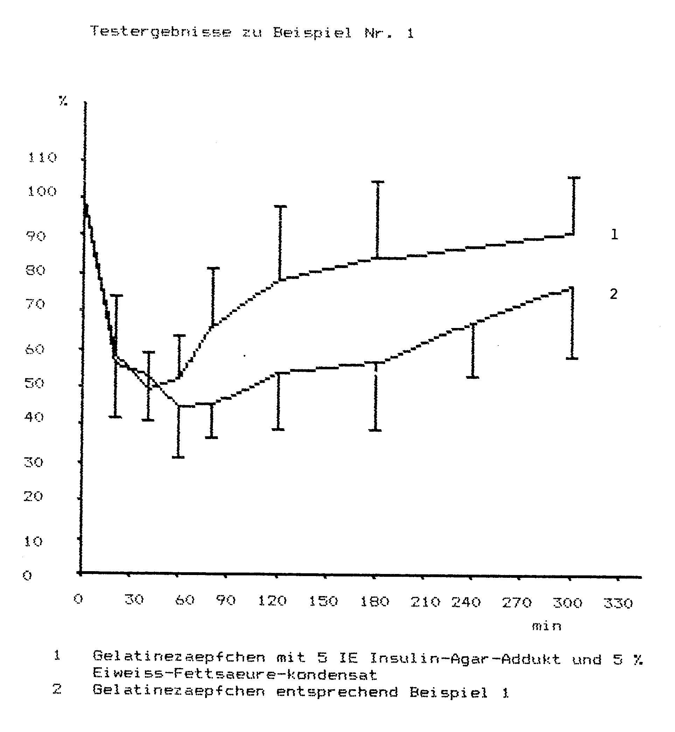

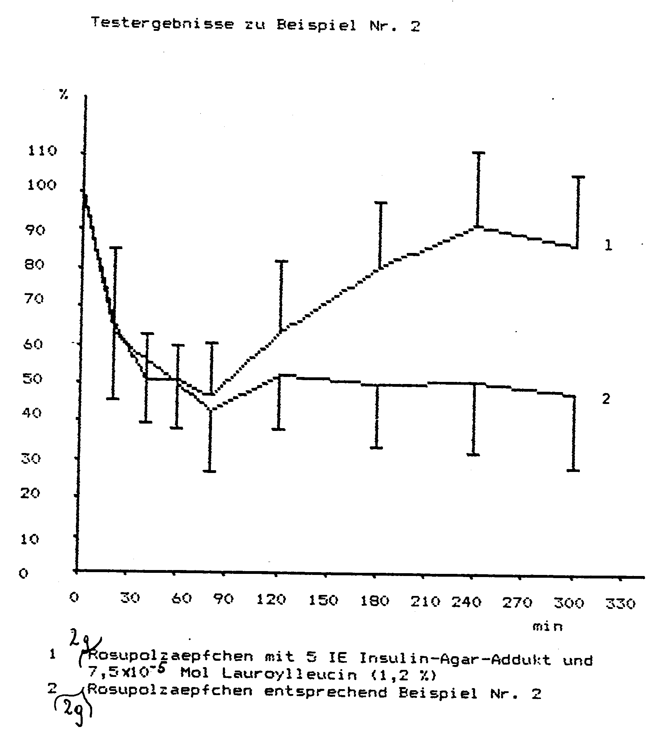

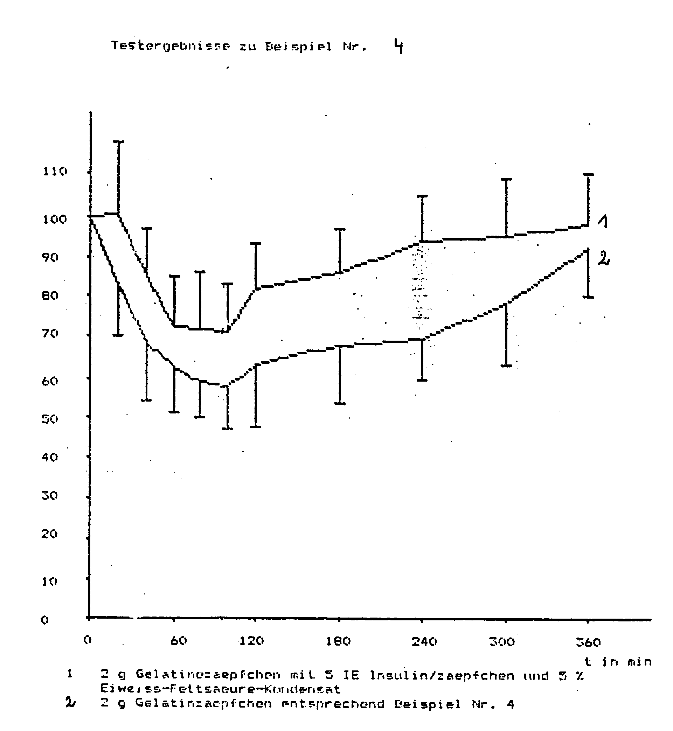

Patentansprüche

Patentansprüche für folgende Vertragsstaaten : DE, FR, GB, IT, SE

1. Arzneimittelpräparat für die rektale und vaginale Anwendung biologisch aktiver Peptide, dadurch gekennzeichnet, dass das Peptid an einen hochmolekularen Träger fixiert ist, mit einem Resorptionsvermittler sowie mit der Kombination eines Proteaseninhibitors mit einem antibakteriellen Wirkstoff und mit weiteren üblichen Träger- und Hilfsstoffen verarbeitet ist.

2. Arzneimittelpräparat nach Anspruch 1, dadurch gekennzeichnet, dass als biologisch aktive Peptide Insuline, GnRH und -Analoga, Substanz P und deren Derivate sowie Teilsequenzen und andere synthetisierte Oligopeptide wie Thymuspeptide, aber auch mikrobiologisch hergestellte Peptide, wie Cyclosporin, eingesetzt sind.

3. Arzneimittelpräparat nach Anspruch 1 und 2, dadurch gekennzeichnet, dass als Resorptionsvermittler anionische Aminosäure-, Peptid- oder Eiweiss-Fettsäure-Kondensate, als Proteaseninhibitor natürliche oder auf synthetischem Wege gewonnene Stoffe, wie Aprotinin oder ε-Aminocapronsäure, und als antibakterieller Wirkstoff Konservierungsmittel oder Chemotherapeutika mit Wirkung gegen anaerobe Bakterien eingesetzt sind.

4. Arzneimittelpräparat nach Anspruch 1 bis 3, dadurch gekennzeichnet, dass als hochmolekularer Träger Polyaminosäuren, Agar-Agar, Dextran, Sephadex oder Agarose eingesetzt sind.

**5.** Verfahren zur Herstellung von Arzneimittelpräparaten gemäss Anspruch 1 bis 4 für die rektale und vaginale Anwendung biologisch aktiver Peptide, dadurch gekennzeichnet, dass das Peptid an einem hochmolekularen Träger fixiert, mit einem Resorptionsvermittler sowie mit der Kombination eines Proteaseninhibitors mit einem antibakteriellen Wirkstoff und mit weiteren üblichen Träger- und Hilfsstoffen zur Arzneiform verarbeitet wird.

**6.** Verfahren nach Anspruch 5, dadurch gekennzeichnet, dass als biologisch aktive Peptide Insuline, GnRH und -Analoga, Substanz P und deren Derivate sowie Teilsequenzen und andere synthetisierte Oligopeptide, wie Thymuspeptide, aber auch mikrobiologisch hergestellte Peptide, wie Cyclosporine, eingesetzt werden.

**7.** Verfahren nach Anspruch 5 und 6, dadurch gekennzeichnet, dass als hochmolekularer Träger Polyaminosäuren, Agar-Agar, Dextran, Sephadex und Agarose eingesetzt werden.

**8.** Verfahren nach Anspruch 5 bis 7, dadurch gekennzeichnet, dass als Resportionsvermittler anionische Aminosäure-, Peptid- oder Eiweiss-Fettsäure-Kondensate eingesetzt werden.

**9.** Verfahren nach Anspruch 5 bis 8, dadurch gekennzeichnet, dass als Proteaseninhibitor natürlich oder auf synthetischem Wege gewonnene Stoffe, wie Aprotinin oder $\epsilon$-Aminocapronsäure, eingesetzt werden.

**10.** Verfahren nach Anspruch 5 bis 9, dadurch gekennzeichnet, dass als antibakterieller Wirkstoff Konservierungsmittel oder Chemoterapeutika mit Wirkung gegen anaerobe Bakterien eingesetzt werden.

**11.** Verfahren nach Anspruch 5 bis 10, dadurch gekennzeichnet, dass als Applikationsform Suppositorien, Kugeln, Kapseln oder Lösungen und Suspensionen hergestellt werden.

**12.** Verfahren nach Anspruch 5 bis 11, dadurch gekennzeichnet, dass die Applikationsform in lipophiler oder hydrophiler Form hergestellt wird.

**Patentansprüche für folgenden Vertragsstaat : ES**

**1.** Verfahren zur Herstellung von Arzneimittelpräparaten für die rektale und vaginale Anwendung biologisch aktiver Peptide, dadurch gekennzeichnet, dass das Peptid an einem hochmolekularen Träger fixiert, mit einem Resorptionsvermittler sowie mit der Kombination eines Proteaseninhibitors mit einem antibakteriellen Wirkstoff und mit weiteren üblichen Träger- und Hilfsstoffen zur Arzneiform verarbeitet wird.

**2.** Verfahren nach Anspruch 1, dadurch gekennzeichnet, dass als biologisch aktive Peptide Insuline, GnRH und -Analoga, Substanz P und deren Derivate sowie Teilsequenzen und andere synthetisierte Oligopeptide, wie Thymuspeptide, aber auch mikrobiologisch hergestellte Peptide, wie Cyclosporine, eingesetzt werden.

**3.** Verfahren nach Anspruch 1 und 2, dadurch gekennzeichnet, dass als hochmolekularer Träger Polyaminosäuren, Agar-Agar, Dextran, Sephadex und Agarose eingesetzt werden.

**4.** Verfahren nach Anspruch 1 bis 3, dadurch gekennzeichnet, dass als Resportionsvermittler anionische Aminosäure-, Peptid- oder Eiweiss-Fettsäure-Kondensate eingesetzt werden.

**5.** Verfahren nach Anspruch 1 bis 4, dadurch gekennzeichnet, dass als Proteaseninhibitor natürlich oder auf synthetischem Wege gewonnene Stoffe, wie Aprotinin oder $\epsilon$-Aminocapronsäure, eingesetzt werden.

**6.** Verfahren nach Anspruch 1 bis 5, dadurch gekennzeichnet, dass als antibakterieller Wirkstoff Konservierungsmittel oder Chemoterapeutika mit Wirkung gegen anaerobe Bakterien eingesetzt werden.

**7.** Verfahren nach Anspruch 1 bis 6, dadurch gekennzeichnet, dass als Applikationsform Suppositorien, Kugeln, Kapseln oder Lösungen und Suspensionen hergestellt werden.

**8.** Verfahren nach Anspruch 1 bis 7, dadurch gekennzeichnet, dass die Applikationsform in lipophiler oder hydrophiler Form hergestellt wird.

**Claims**

**Claims for the following Contracting States : DE, FR, GB, IT, SE**

**1.** Medicament preparation for rectal and vaginal administration of biologically active peptides, characterised in that the peptide is fixed to a high molecular substrate, is processed with a resorption promoter and with the combination of a protease inhibitor and an anti-bacterial active ingredient and with further conventional excipients and auxiliaries.

**2.** Medicament preparation according to claim 1, characterised in that insulins, GnRH and GnRH analogues, substance P and derivatives thereof and partial sequences and other synthesised oligopeptides, such as thymus peptides, but also microbiologically produced peptides, such as cyclosporin, are used as biologically active peptides.

**3.** Medicament preparation according to claim 1 and 2, characterised in that anionic amino acid; peptide- or protein-fatty acid condensates, are used as resorption promoters, natural substances or substances obtained by synthetic means, such as aprotinin or $\epsilon$-aminocaproic acid, are used as protease inhibitors, and preservatives or chemotherapeutic agents having an effect against anaerobic bacteria are used as anti-bacterial active ingredient.

**4.** Medicament preparation according to claim 1 to 3, characterised in that polyamino acids, agar-agar, dextran, Sephadex or agarose are used as high molecular substrates.

**5.** Process for producing medicament preparations according to claim 1 to 4 for rectal and vaginal administration of biologically active peptides, characterised in that the peptide is fixed to a high molecular substrate, is processed with a resorption promoter and with the combination of a protease inhibitor and an anti-bacterial active ingredient and with further conventional excipients and auxiliaries to give the preparation.

**6.** Process according to claim 5, characterised in that insulins, GnRH and GnRH analogues, substance P and derivatives thereof and partial sequences and other synthesised oligopeptides, such as thymus peptides, but also microbiologically produced peptides, such as cyclosporins, are used as biologically active peptides.

**7.** Process according to claim 5 and 6, characterised in that polyamino acids, agar-agar, dextran, Sephadex and agarose, are used as high molecular substrates.

**8.** Process according to claim 5 to 7, characterised in that anionic amino acid, peptide or protein-fatty acid condensates are used as resorption promoters.

**9.** Process according to claim 5 to 8, characterised in that substances obtained naturally or by synthetic means, such as aprotinin or $\epsilon$-aminocaproic acid, are used as protease inhibitors.

**10.** Process according to claim 5 to 9, characterised in that preservatives or chemotherapeutic agents having an effect against anaerobic bacteria are used as anti-bacterial active ingredient.

**11.** Process according to claim 5 to 10, characterised in that suppositories, beads, capsules or solutions and suspensions are produced as a form of administration.

**12.** Process according to claim 5 to 11, characterised in that the form of administration is produced in lipophilic or hydrophilic form.

**Claims for the following Contracting State : ES**

**1.** Process for producing medicament preparations for rectal and vaginal administration of biologically active peptides, characterised in that the peptide is fixed to a high molecular substrate, is processed

8

with a resorption promoter and with the combination of a protease inhibitor and an anti-bacterial active ingredient and with further conventional excipients and auxiliaries to give the preparation.

2. Process according to claim 1, characterised in that insulins, GnRH and GnRH analogues, substance P and derivatives thereof and partial sequences and other synthesised oligopeptides, such as thymus peptides, but also microbiologically produced peptides, such as cyclosporins, are used as biologically active peptides.

3. Process according to claim 1 and 2, characterised in that polyamino acids, agar-agar, dextran, Sephadex and agarose, are used as high molecular substrates.

4. Process according to claim 1 to 3, characterised in that anionic amino acid, peptide-or protein-fatty acid condensates are used as resorption promoters.

5. Process according to claim 1 to 4, characterised in that substances obtained naturally or by synthetic means, such as aprotinin or $\epsilon$-aminocaproic acid, are used as protease inhibitors.

6. Process according to claim 1 to 5, characterised in that preservatives or chemotherapeutic agents having an effect against anaerobic bacteria are used as anti-bacterial active ingredient.

7. Process according to claim 1 to 6, characterised in that suppositories, beads, capsules or solutions and suspensions are produced as a form of administration.

8. Process according to claim 1 to 7, characterised in that the form of administration is produced in lipophilic or hydrophilic form.

**Revendications**
**Revendications pour les Etats contractants suivants : DE, FR, GB, IT, SE**

1. Préparation médicamenteuse pour l'utilisation rectale et vaginale de peptides biologiquement actifs, caractérisée en ce que le peptide est fixé sur un support de masse moléculaire élevée, est mis en oeuvre avec un agent favorisant la résorption ainsi qu'avec l'association d'un inhibiteur des protéases et d'une substance active antibactérienne, et avec d'autres supports et adjuvants habituels.

2. Préparation médicamenteuse selon la revendication 1, caractérisée en ce qu'on utilise comme peptides biologiquement actifs des insulines, le GnRH et ses analogues, la substance P et ses dérivés ainsi que des séquences partielles et d'autres oligopeptides de synthèse comme les peptides du thymus, mais aussi des peptides préparés par voir microbiologique, tels que la cyclosporine.

3. Préparation médicamenteuse selon les revendications 1 ou 2, caractérisée en ce qu'on utilise comme agent favorisant la résorption des condensats anioniques, aminoacide -, peptide- ou protéine-acide gras, comme inhibiteur de protéases des substances naturelles ou obtenues par voie synthétique telles de l'aprotinine ou l'acide epsilon -aminocaproïque et comme substance active antibactérienne des conservateurs ou des agents chimiothérapiques actifs contre les bactéries anaérobies.

4. Préparation médicamenteuse selon les revendications 1 à 3, caractérisée en ce qu'on utilise comme support de masse moléculaire élevée des polyaminoacides, la gélose, le dextrane, le Sephadex ou l'agarose.

5. Procédé pour la fabrication de préparations médicamenteuses selon les revendications 1 à 4 pour l'utilisation rectale et vaginale de peptides biologiquement actifs, caractérisé en ce qu'on fixe le peptide sur un support de masse moléculaire élevée, et on le transforme en une forme médicamenteuse avec un agent favorisant la résorption ainsi qu'avec une association d'un inhibiteur de protéases avec une substance active antibactérienne et avec d'autres supports et adjuvants habituels.

6. Procédé selon la revendication 5, caractérisé en ce qu'on utilise comme peptides biologiquement actifs des insulines, le GnRH et ses analogues, la substance P et ses dérivés et leurs séquences partielles ainsi que d'autres oligopeptides préparés par synthèse tels que les peptides du thymus, mais aussi des peptides préparés par voie microbiologique tels que les cyclosporines.

7. Procédé selon les revendications 5 et 6, caractérisé en qu'on utilise comme support de masse moléculaire élevée des polyaminoacides, la gélose, le dextrane, le Sephadex et l'agarose.

8. Procédé selon les revendications 5 à 7, caractérisé en ce qu'on utilise comme agent favorisant la résorption des condensats anioniques aminoacide -, peptide-ou protéine-acide gras.

9. Procédé selon les revendications 5 à 8, caractérisé en ce qu'on utilise comme inhibiteur des protéases des substances obtenues naturellement ou par voie synthétique telles que l'aprotinine ou l'acide epsilon - aminocaproïque.

10. Procédé selon les revendications 5 à 9, caractérisé en ce qu'on utilise comme substance active antibactérienne des conservateurs ou des produits chimiotérapiques actifs contre les bactéries anaérobies.

11. Procédé selon les revendications 5 à 10, caractérisé en ce qu'on prépare comme forme d'administration des suppositoires, des sphères, des capsules ou des solutions et des suspensions.

12. Procédé selon les revendications 5 à 11, caractérisé en ce que la forme d'administration est préparée sous forme lipophile ou hydrophile.

**Revendications pour l'Etat contractant suivant : ES**

1. Procédé de fabrication de préparations médicamenteuses pour l'administration rectale et vaginale de peptides biologiquement actifs, caractérisé en ce que le peptide est fixé sur un support de masse moléculaire élevée, et transformé en forme médicamenteuse avec un agent favorisant la résorption ainsi qu'avec l'association d'un inhibiteur des protéases avec une substance active antibactérienne, et avec d'autres supports et adjuvants habituels.

2. Procédé selon la revendication 1, caractérisé en ce qu'on utilise comme peptides biologiquement actifs des insulines, le GnRH et ses analogues, la substance P ainsi que leurs dérivés et séquences partielles et d'autres oligopeptides préparés par synthèse tels les peptides du thymus, mais aussi des peptides préparés par voie microbiologique, tels que la cyclosporine.

3. Procédé selon les revendications 1 et 2, caractérisé en ce qu'on utilise comme support de masse moléculaire élevée des polyaminoacides, la gélose, le dextrane, le Sephadex, et l'agarose.

4. Procédé selon les revendications 1 à 3, caractérisé en ce qu'on utilise comme agent favorisant la résorption des condensats anioniques aminoacide-, peptide-ou protéine-acide gras.

5. Procédé selon les revendications 1 à 4, caractérisé en ce que l'on utilise comme inhibiteur des protéases des substances obtenues naturellement ou par voie synthétique telles de l'aprotinine ou l'acide epsilonaminocaproïque.

6. Procédé selon les revendications 1 à 5, caractérisé en ce qu'on utilise comme substances actives anti-bactériennes des conservateurs ou des produits chimiothérapiques actifs contre les bactéries anaérobies.

7. Procédé selon les revendications 1 et 6, caractérisé en qu'on prépare comme forme d'administration des suppositoires, des sphères, des capsules ou des solutions et des suspensions.

8. Procédé selon les revendications 1 à 7, caractérisé en ce que la forme d'administration est préparée sous forme lipophyle ou hydrophile.

Testergebnisse zu Beispiel Nr. 1

1   Gelatinezaepfchen mit 5 IE Insulin-Agar-Addukt und 5 %
     Eiweiss-Fettsaeure-kondensat
2   Gelatinezaepfchen entsprechend Beispiel 1

Testergebnisse zu Beispiel Nr. 2

1 Rosupolzaepfchen mit 5 IE Insulin-Agar-Addukt und
  7,5x10$^{-6}$ Mol Lauroylleucin (1,2 %)
2 Rosupolzaepfchen entsprechend Beispiel Nr. 2

12

Testergebnisse zu Beispiel Nr.  4

1   2 g Gelatinezaepfchen mit 5 IE Insulin/zaepfchen und 5 %
    Eiweiss-Fettsaeure-Kondensat
2   2 g Gelatinzaepfchen entsprechend Beispiel Nr. 4